# NEUE EUROPÄISCHE PATENTSCHRIFT

(11) **EP 0 176 720 B2**
(45) Veröffentlichungstag und Bekanntmachung des Hinweises auf die Entscheidung über den Einspruch: **09.03.1994**
(45) Hinweis auf die Patenterteilung: 18.04.1990
(21) Anmeldenummer: 85110268.1
(22) Anmeldetag: 16.08.1985
(51) Int. Cl.: A01N 31/02, A01N 65/00, A61K 31/045

(54) **Viruzides Mittel**
Anti-viral agent
Agent antiviral

(30) Priorität: 21.08.1984 DE 3430709
(43) Veröffentlichungstag der Anmeldung: 09.04.1986
(73) Patentinhaber: Krüger GmbH & Co. KG, D-51435 Bergisch Gladbach (DE)
(72) Erfinder: Eggers, Hans J., Prof. Dr., D-5000 Köln 41 (DE); Schott, Gerhard, D-4020 Mettmann (DE)
(74) Vertreter: Werner, Hans-Karsten, Dr.Dipl.-Chem.

(56) Entgegenhaltungen:
- EP-A- 0 030 576
- DE-A- 2 333 849
- DE-A- 3 117 792
- CHEMICAL ABSTRACTS, Band 92, Nr. 16, 21. April 1980, Seite 404, Nr. 135140r, Columbus, Ohio, US; & JP - A - 79 132 241 (DAISAN GOMAI K.K.) 15.10.1979
- DICTIONNAIRE FRANCAIS DE MEDECINE ET DE BIOLOGIE, Band 1, 1970, Seiten 109-110, Paris, FR; A. MANUILA et al.
- THE MERCK INDEX, Band 9, 1976, Seite 242, Rahway, New Yersey, US; Binder et al.
- M G de Navarre "The Chemistry and Manufacture of Cosmetics", Vol II (1962), S.165-166
- J S Jellinek "Kosmetologie", 2. Aufl., S.402-416

## Beschreibung

Gegenstand der vorliegenden Erfindung ist die Verwendung eines Gemisches zur Herstellung eines viruziden Mittels gegen nackte Viren, zu denen man unter anderem Poliovirus 1, Echovirus 11, Echovirus 12, Rotavirus, Reovirus 3 und Adenovirus 2 zählt.

Aus der DE-A-3117792 ist die Verwendung einer wäßrigen Lösung mit einem Gehalt von 3 bis 55 Gew.- % eines ein- oder mehrwertigen Akohols mit biz zu 8 C-Atomen bekannt. Die Lösung soll insbesondere gegen Heptatitis B und Papovaviren für die Haut-, Hände-, Flächen- und Instrumentaldesinfektion geeignet sein.

Die EP-A-0030576 beschreibt eine filmbildende mikrobizide alkoholische Zusammensetzung zum Eintauchen von Zitzen und Verfahren zu deren Verwendung. Als Mikrobizide werden niedere Alkohole n einem Konzentrationsbereich von 15 bis 70% verwendet, die gegen Mastitis wirksam sind.

Die DE-A-2333849 beschreibt ein mikrobizides Mittel bestehend aus niederen Alkoholen, Benzylalkoholderivaten, Wasser sowie nichttoxischen, pharmazeutisch verträglichen Trägern und Formulierungsstoffen, das insbesondere gegen behüllte Viren wirksam ist.

Die rote Liste Nummer 32058 (1987) beschreibt eine als AHD 2000 bezeichnete Lösung enthaltend Ethanol, Rückfetter sowie tierische Öle, die zur chirurgischen Hände- und Haut-Desinfektion jedoch nicht zur Inaktivierung von nackten Viren geeignet ist.

Es ist weiterhin bekannt, daß Viren mit einer Lipoidhülle relativ empfindlich sind und daher auch mit bischer bekannten viruziden Desinfektionsmitteln inaktiviert bzw. sogar abgetötet werden können. Problematisch und wesentlich beständiger gegen übliche Desinfektionsmittel sind hingegen die nackten Viren, die sich bischer eigentlich nur mit relativ hohen Konzentrationen von Formaldehyd töten bzw. inaktivieren lassen. Formaldehyd ist jedoch aus toxikologischen Gründen unerwünscht und gestattet weder in der Klinik noch im Labor die Desinfektion von infizierten Körperteilen.

Die Literatur über die desinfizierende bzw. über die inaktivierende Wirksamkeit von handelsüblichen, hautverträglichen Desinfektionsmitteln ist widersprüchlich. Eigene Untersuchungen haben jedoch ergeben, daß die meisten hautverträglichen Desinfektionsmittel gegen nackte Viren praktisch unwirksam sind. Auch die Angaben über die Wirksamkeit von Alkoholen sind widersprüchlich. Eigene Untersuchungen über die Wirksamkeit von Isopropanol gegen nackte Viren haben gezeigt, daß dieser Alkohol nur sehr schwach wirksam oder unwirksam gegen nackte Viren ist und daher ebenfalls ungeeignet ist. Diese Untersuchungen haben weiterhin ergeben, daß überraschenderweise Ethanol und Methanol hoch wirksam sind und ihre Wirksamkeit erst verlieren, wenn sie mehr als 30% Wasser enthalten. Während Ethanol gegen Baktieren und Pilze auch noch in wesentlichen geringeren Konzentrationen desinfizierend bzw. inaktivierende wirkt muß somit für die Abtötung von nackten Viren der Wassergehalt der Alkohole wesentlich geringer sein.

Da alle Alkohole bei der Anwendung auf der Haut zu einer unerwünschten Entfettung führen und die Haut spröde und rissig machen, lag es nahe, des Alkoholen hautschonende Zusätze zuzufügen, die eine Rückfettung bewirken. Entsprechende Untersuchungen ergaben, daß überraschenderweise Zusätze von 1 bis 10% Glycerol zu Methanol und Ethanol die Wirksamkeit dieser Alkohole nicht herabgesetzt haben. Weiterhin wurde gefunden, daß Zusätze bis zu 5% Ricinusöl ebenfalls die Aktivität gegenüber nackten Viren nicht verschlechtern, jedoch zur besseren Hautverträglichkeit beitragen.

Gegenstand der vorliegenden Erfindung ist somit die Verwendung enes Gemisches aus mindestens 70% Methanol und/oder Ethanol mit 1 bis 10% Glycerol sowie 0,5. bis zu 5% Rizinusöl zur Herstellung eines viruziden Mittels zur Desinfektion und/oder Inaktivierung von nackten Viren.

Da bei Konzentrationen der Alkohole unter 70% die Wirksamkeit rasch abfällt, enthält das erfindungsgemäß hergestellte viruzide Mittel bis zu 97% vorzugsweise 80 bis 90% Methanol und/oder Ethanol. Die Mengen an Glycerol betragen vorzugsweise 1 bis 5% und die Zusätze an Ricinusöl vorzugsweise 0,5 bis 2%.

Prinzipiell ist es möglich, diesen Mitteln noch weitere hautverträgliche Zusätze zuzugeben, jedoch ist der Zusatz von Triglyceriden zu vermeiden, da diese die Aktivität stark herabsetzen. Unter Umständen sind es auch die von reinen Alkoholen gelösten Hautfette gewesen, die bei früheren Untersuchungen zu dem Ergebnis geführt haben, daß die einfachen Alkohole wie Methanol und Ethanol zurAbtötung bzw. zur Inaktivierung von nackten Viren ungeeignet sind.

Der Zusatz von Wasser zum Methanol und Ethanol ist undschädlich, solange eine ausreichend hohe Konzentration an Methanol oder Ethanol gewährleistet ist.

Die Wirksamkeit des erfindungsgemäß hergestellten viruziden Mittels ist bisher wissenschaftlich nicht erklärbar. Die Vermutung, daß die Wirkung allein auf einer Entwässerung beruht, kann nicht zutreffen, da ebenfalls starkentwässernde Lösungsmittel wie Propylakohol, Isopropylalkohol und Aceton unwirksam sind.

Die Entscheidung, ob die erfindungsgemäß hergestellten Mittel Methanol, Ethanol oder Gemische der beiden Alkohole enthalten, hängt von den Kosten, der Toxizität und etwaiger gesetzlicher Regelungen über den gewerblichen Gebrauch der Alkohole ab. So ist Methanol zwar noch etwas wirksamer als Ethanol, jedoch hat es eine höhere Toxizität als Ethanol. Ethanol hingegen ist teurer und unterliegt obendrein in vielen Ländern gesetzlichen Spezialvorschriften, die seinen Einsatz für die Herstellung der erfindungsgemäß hergestellten Mittel beeinträchtigen können. Da die Wirksamkeit von Gemischen von Methanol und Ethanol linear mit steigenden Anteilen von Methanol ansteigt, können, ohne weiteres aush Gemische der beiden Alkohole eingesetzt werden und somit Kompromisse zwischen der Vor- und Nachteilen der beiden Alkohole erreicht werden.

Die erfindungsgemäß hergestellten Mittel sind so wirksam, daß sie innerhalb von einer Minute, spätestens jedoch nach 15 Minuten die Aktivität und Infektiosität der häufigsten nackten Viren um mindestens 4 Zehnerpotenzen reduzieren.

Bei der Anwendung der erfindungsgemäß hergestellten Mittel können diese entweder als Spülmittel verwendet werden oder mit Hilfe von Watte, Stofflappen oder ähnlichen Hilfsmitteln auf die infizierten Hautteile aufgebracht werden. Wegen der inaktivierenden Wirkung von Triglyceriden ist es dabei zu empfehlen, zunächst bei einer ersten Anwendung die Hautfette zu entfernen und dann das erfindungsgemäß hergestellte Mittel bei einer zweiten Anwendung zur Einwirkung zu bringen. Der Gehalt an Glycerin und Ricinusöl verhindert eine zu starke und unerwünschte Austrocknung der Haut und wirkt daher in ähnlicher Weise wie eine an sich erwünschte sofortige Rückfettung. Im übrigen ist es durchaus möglich, nach der Verwendung der neuen Mittel die Haut mit geeigneten, Triglycerid enthaltenden Hautpflegemitteln zu behandeln, da die geforderte Desinfektion bzw. Inaktivierung in Abwensenheit der Triglyceride erfolgt ist.

Aus den nachfolgenden Beispielen und Vergleichversuchen geht die Zusammensetzung und die Wirksamkeit der erfindungsgemäß hergestellten viruziden Mittel hervor.

### Beispiel 1

95 Volumenteile 96%-iger Ethanol werden mit 4 Vol-% Ricinusöl und 1 Vol-% Glycerol vermischt. In einem zweiten Ansatz wird dieselbe Menge Ethanol mit 1 % Ricinusöl und 4% Glycerol versetzt. In einer weiteren Rezeptur werden 97 Volumenteile Methanol jeweils 1 % Glycerol und 2% Ricinusöl oder 3% Glycerol und 1% Ricinusöl zugegeben. Untersuchungen mit Poliovirus 1 (Mahoney) im Suspensionsversuch bei 20°C zeigen, daß nach 1 bis 5 Minuten die Infektionsität um 3 bis 5 Zehnerpotenzen absinkt.

Vergleichsversuche mit entsprechenden Lösungen von Aceton, Isopropanol und Propanol zeigten keine meßbare Wirkung.

Durch Zusatz von steigenden Mengen Wasser in den Gemischen wurde festgestellt, daß Gemische mit mindestens 70 Vol.-% Methanol und/ oder Ethanol noch stark wirksam sind. Gemische mit nur noch 50% Methanol oder Ethanol sind bereits unwirksam.

Vergleichsversuche mit Methanol und Ethanol unter Zusatz von 1 bis 2% Triglyceriden zeigten, daß diese Gemische nur noch sehr schwach wirksam waren.

### Beispiel 2

Hautpartien wurden zum Vergleich mit reinem Methanol und reinem Ethanol sowie wäßrigem Methanol und wäßrigem Ethanol abgerieben. Diese Hauptpartien wurden hierdurch in bekannter Weise völlig entfettet und trocken. Vergleichsversuche mit entsprechenden Alkoholen unter Zusatz von Glycerin und Ricinusöl zeigten, daß die Haut auf nach der Behandlung zwar entfettet war, aber feucht blieb.

## Patentansprüche

1. Verwendung eines Gemisches aus mindestens 70% Methanol und/oder Ethanol mit 1 bis 10% Glycerol sowie 0,5. bis zu 5% Rizinusöl zur Herstellung eines viruziden Mittels zur Desinfektion und/oder Inaktivierung von nackten Viren.

## Revendications

1. Utilisation d'un mélange constitué d'au moins 70% de méthanol et/ou d'éthanol avec 1 à 10% de glycérine ainsi que 0,5 à 5% d'huile de ricin pour la fabrication d'un agent antiviral pour la désinfection et/ou l'inactivation de virus nus.

## Claims

1. Use of a mixture comprising at least 70% of methanol and/or ethanol with 1 to 10% of glycerol and from 0,5 to 5% of castor oil forthe preparation of a virucidal agent for disinfecting and/or inactivation of naked viruses.
